# EUROPEAN PATENT APPLICATION

(11) **EP 3 492 051 A1**
(43) Date of publication of application: **05.06.2019**
(21) Application number: 18208117.4
(22) Date of filing: 23.11.2018
(51) Int. Cl.: A61F 5/01

(54) **ARTICULATED ROD OF AN ORTHOPAEDIC DEVICE**

(30) Priority: 01.12.2017 IT 201700139050
(71) Applicant: Orthoservice AG, 6830 Chiasso (CH)
(72) Inventor: TURCONI, Francesco, 20900 Monza (MB) (IT); ALBERIO, Daniele, 23898 Imbersago (LC) (IT)
(74) Representative: Branca, Emanuela

(57) **Abstract**

Articulated rod (10) of an orthopaedic device comprising a pair of arms (20) in mutual flexion-extension rotation, two cover plates (30), a polycentric joint (40), two rotation pins (42) engaged in through holes (34) of at least one cover plate (32) and in through holes (25) of said pair of arms (20), each arm (20) bears a slot (29), a pair of cams (50) rotatably engaged in through openings (35) of said at least one cover plate (31), each cam (50) bears a through hole (55) engaged by a rotation pin (42), comprising at least one axial extension (59) adapted to be engaged in the slot (29), a plurality of coupling teeth (56) and an indication element (54), at least one locking element (60, 70) comprising a plurality of teeth (65) adapted to be engaged with said plurality of coupling teeth (56).

## Description

The present invention refers to an articulated rod of an orthopaedic device.

In the state of the art there are articulated rods of orthopaedic devices with polycentric joints and removable inserts for constraining a flexion-extension rotation within a certain width. It is necessary to use inserts of different shapes and sizes to constrain different rotation widths. Disadvantageously, whenever the rotation width is to be changed, it is necessary to dismount the inserts from the joint and replace them with others making the adjustment operation complicated as it is connected with mounting and dismounting the inserts.

Inserts exist that are removable in an orthogonal direction to a rotation plane of arms that constrain the rotation within a slot, but disadvantageously they constrain the movement of both arms together, making it difficult and impractical to adjust the rotation width of the articulated rod.

The object of the present invention is to provide an articulated rod with an easily adjustable flexion-extension rotation, which is secure, compact and small.

According to the present invention said object is achieved by providing an articulated rod as disclosed in claim 1.

Further characteristics are highlighted in the dependent claims.

The characteristics and advantages of an articulated rod with a polycentric joint that can be adjusted in extension-flexion according to the present invention will be more apparent from the following description, which is to be understood as exemplifying and not limiting, with reference to the schematic attached drawings, wherein:
Figure 1 shows an exploded perspective view of the articulated rod with a polycentric joint, arms with through holes and slots, cams, toothed button, stop buttons and cover;
Figure 2 shows a section of the articulated rod with the polycentric joint with arms hinged with the cams;
Figure 3 shows a view of a cam from the outside;
Figure 4 shows a view of the cam from the inside;
Figure 5 shows a view of the toothed button from the outside;
Figure 6 shows a view of the toothed button from the inside;
Figure 7 shows a view of a stop button from the outside;
Figure 8 shows a view of the stop button from the inside;
Figure 9 shows a view of the cover from the outside;
Figure 10 shows a view of the cover from the inside;
Figure 11 shows a view from the outside of the articulated rod with the stop buttons disengaged from the cover and from the toothed button which is in turn disengaged from the cams that are free to rotate on rotation pins;
Figure 12 shows a dismounted detail of the articulated rod with the stop buttons in the disengaged position;
Figure 13 shows a perspective view of Figure 12;
Figure 14 shows a dismounted detail of the articulated rod with the stop buttons in the engaged position with the cover, with the toothed button, which is in turn engaged with the cams, blocking the movement thereof;
Figure 15 shows a perspective view of Figure 14.

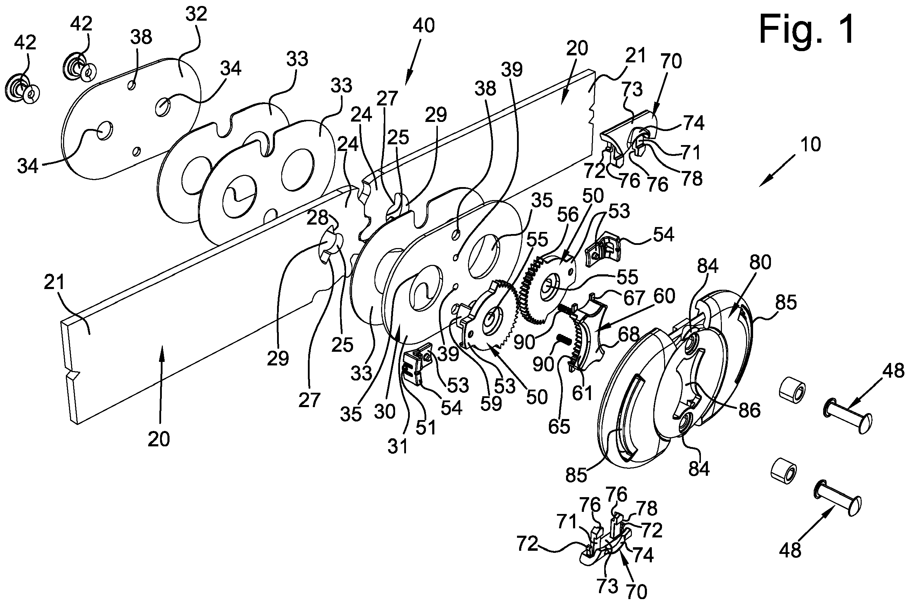

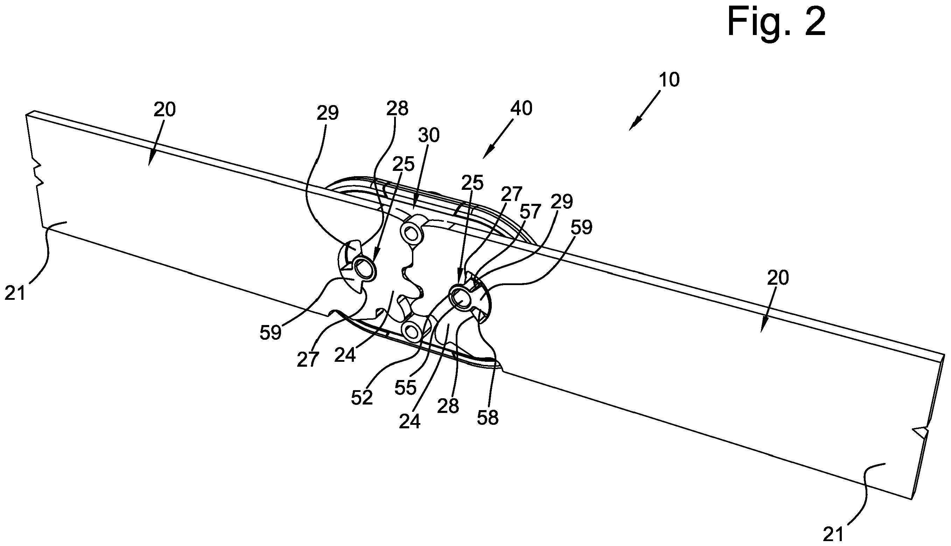

With particular reference to Figures 1 and 2, an articulated rod 10 of an orthopaedic device is shown comprising a pair of arms 20, two cover plates 30 and a polycentric joint 40 that is mounted with the two cover plates 30 and that articulates the pair of arms 20.

The articulated rod 10 further comprises two cams 50, a toothed button 60, two stop buttons 70 and a cover 80.

The two cover plates 30 are an outer cover plate 31 and an inner cover plate 32, where the terms outer and inner refer to mounting directions with respect to the orthopaedic device.

In general the polycentric joint 40 is defined overall by the means that allows the two arms 20 to mesh with the two plates 30. The polycentric joint 40 allows a mutual rotation of the pair of arms 20. The rotation comprises two rotation directions which are a direction of flexion rotation and a direction of extension rotation. The rotation takes place on a geometric plane of rotation.

Each arm 20 bears a first end 21 that can be associated with the orthopaedic device and a toothed end 24 opposite the first end 21.

The toothed ends 24 of the pair of arms 20 are meshed together for performing the rotation. Each toothed end 24 bears a central hole 25 in the axial position with respect to the extension of the toothed profile.

The two cover plates 30 are coupled to each other to form a shell and house the two toothed ends 24 and two rotation pins 42 of the articulated rod 10. The two rotation pins 42 are arranged orthogonally with respect to said geometric plane of rotation and are engaged in respective central holes 25 of the toothed ends 24 and define an axial direction.

The inner cover plate 32 bears two through holes 34 in which each of the two rotation pins 42 is engaged respectively.

The outer cover plate 31 bears two through openings 35 in which the cams 50 are rotatably engaged.

Each cam 50 bears a through hole 55 in which the rotation pin 42 is engaged.

Each toothed end 24 bears a slot 29 that is engaged by an axial extension 59 of cam 50. In the example shown in the figures and in particular in Figure 2, the slot 29 is in communication with the through opening 35 of the outer cover plate 31. The slot 29 of the arm 20 has a first end stop wall 27 and a second end stop wall 28, that constrain the movement of the respective axial extension 59 of the cam 50.

As shown in particular in Figure 2, the two slots 29 have a central symmetry with respect to a geometric centre arranged between the two arms 20 that lies on the geometric plane of rotation. This means that observing Figure 2, the left arm 20 shows that the first end stop wall 27 is at the bottom with respect to Figure 2, the second end stop wall 28 is at the top with respect to Figure 2, while for central symmetry, the arm 20 on the right shows that the first end stop wall 27 is at the top with respect to Figure 2 and the second end stop wall 28 is at the bottom with respect to Figure 2.

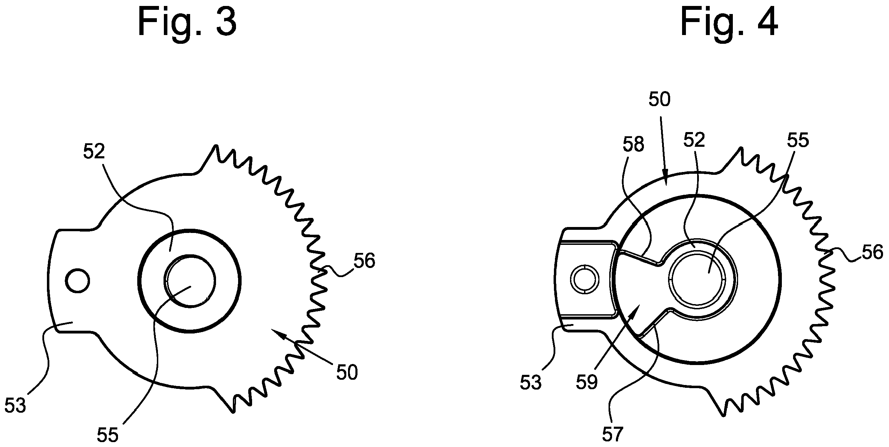

As shown in particular in Figures 3 and 4 each of the two cams 50 is disc-shaped, where the perforated centre of the disc is the through hole 55. For each cam 50 two distinct perimeter geometric profiles can be identified, respectively an outer portion and an inner portion overlapping one another on the geometric plane of rotation and preferably realized as a single piece.

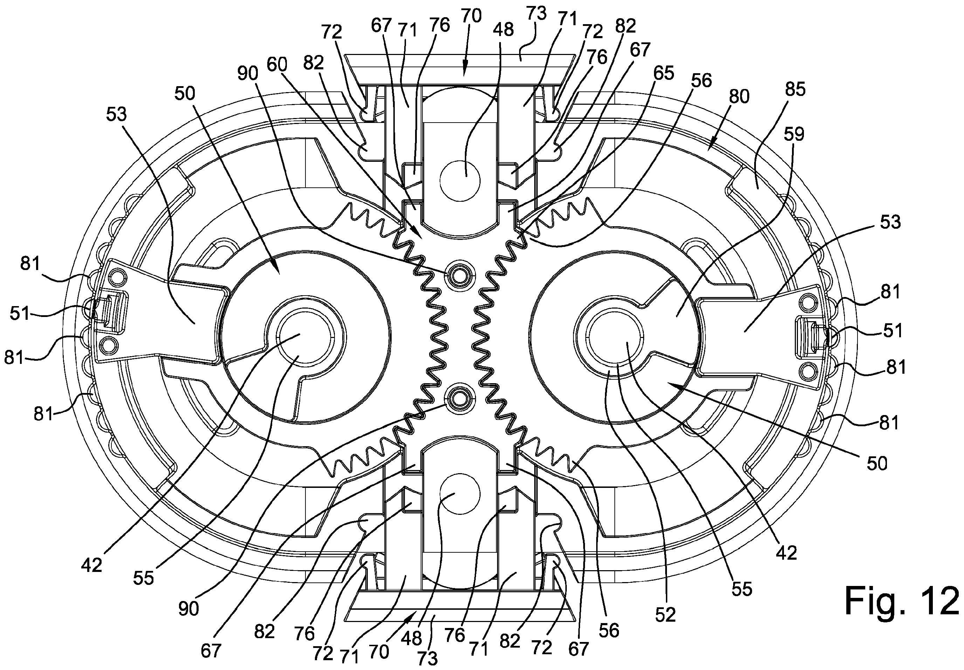

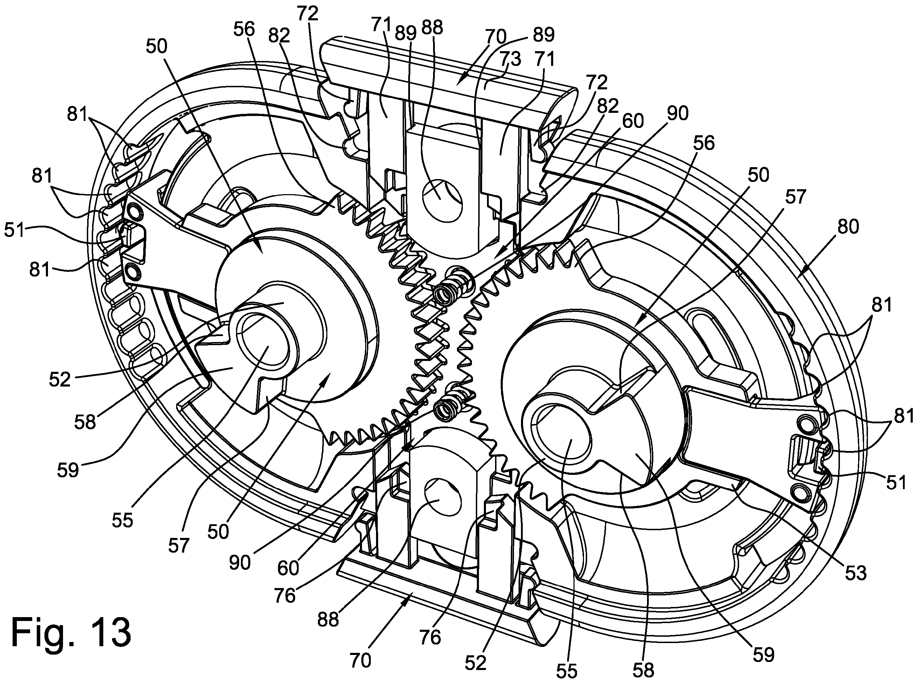

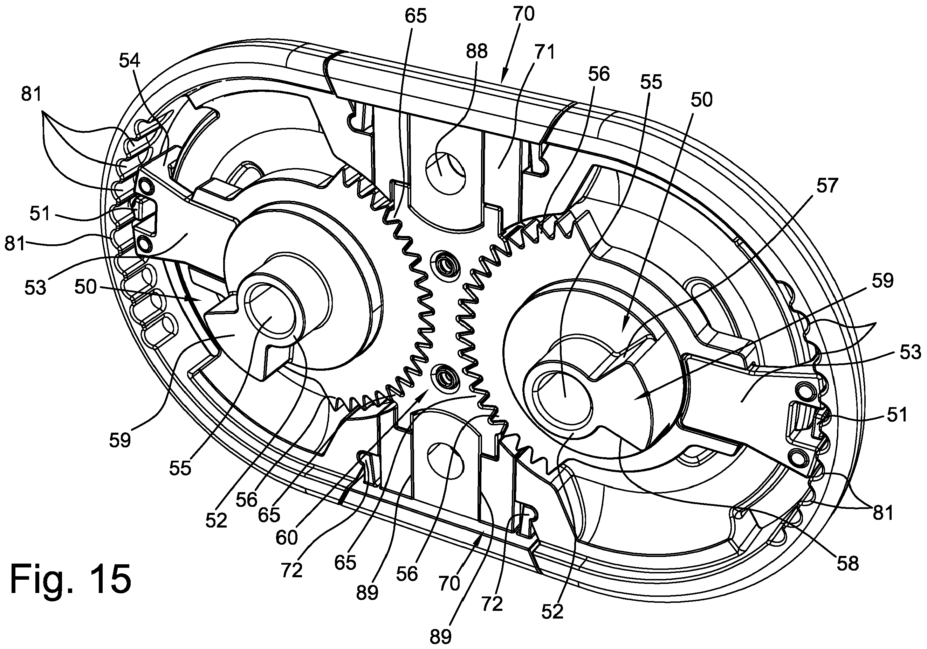

On the geometric plane of rotation it is envisaged that a perimeter segment of the outer portion of the cam 50, extended at about an angular sector of 180°, has a plurality of radial extensions that are coupling teeth 56. A second perimeter segment of the outer portion of the cam 50, diametrically opposite the first perimeter segment, has a radial extension 53. As shown in the embodiment of Figures 1-2, 11-15, it is envisaged that the radial extension 53 of the cam 50 is in two pieces that can be separately mounted with each other. The radial extension 53 of the cam 50 comprises an indication slider 54 that extends in the axial direction and a selector tooth 51 that extends from the wall of the radial extension 53 of the cam 50. The indication slider 54 is a lever adapted to be managed by the orthopaedist.

As shown in particular in Figures 1-4 and 12-15, the inner portion of the cam 50 has a hollow axial extension 52 that identifies the through hole 55 of the cam 50, comprising a succession of discs that extend axially from the outer portion of the cam 50. The succession of discs of the hollow axial extension 52 comprises an outer disc adapted to be rotatably engaged with the through opening 35 of the outer cover plate 31 and an inner disc adapted to be rotatably engaged with the through hole 25 of the arm 20, inner and outer meaning the mounting directions of the articulated rod 10 with the orthopaedic device.

The axial extension 59 engaged in the slot 29 is a single piece with the hollow axial extension 52 of the cam 50. The axial extension 59 of the cam 50 comprises a first stop wall 57 adapted to go into abutment with the first end stop wall 27 of the slot 29 of the arm 20 and a second stop wall 58 adapted to go into abutment with the second stop wall 28 of the slot 29 of the arm 20. In the example described in the figures the axial extension 59 of the cam 50 has a section in the rotation plane in the form of a circular crown for advantageously making the cam 50 and its connection to the slot 29 of the arm 20 more solid.

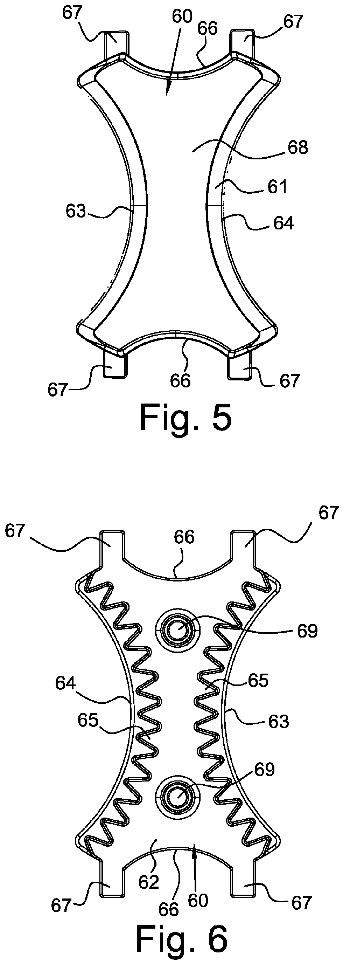

As shown in particular in Figures 5-6, the toothed button 60 comprises two side walls 63, 64 that each comprise a respective plurality of teeth 65 adapted to be engaged by at least one tooth 56 of the pair of cams 50, as shown in Figures 1 and 2. The side wall 63, 64 and the teeth 65 of the toothed button 60 have a complementary shape with respect to the toothed perimeter segment of the cam 50 and the respective teeth 56 of the cam 50 for being engaged together. A first side wall 63 of the toothed button 60 is directed in the direction of the plurality of teeth 56 of a first cam 50 of the two cams 50. A second side wall 64 of the toothed button 60 is directed in the direction of the plurality of teeth 56 of a second cam 50 of the two cams 50.

The button 60 is mounted with the outer cover plate 31 by means of two elastic elements 90 of the articulated rod 10 so as to be able to pass from an engagement position to a disengagement position with the cams 50.

In the embodiment shown in the figures, the elastic element 90 is a helical spring. The elastic element 90 passes from a work position where the helical spring 90 is compressed to a relaxed position where the helical spring 90 is elongated. The toothed button 60 has an outer face 61 and an inner face 62. The inner face 62 bears two seats 69 each adapted to house a respective end of the elastic element 90. The other end of the elastic element 90 is housed in a respective seat 39 of an outer face of the outer cover plate 32.

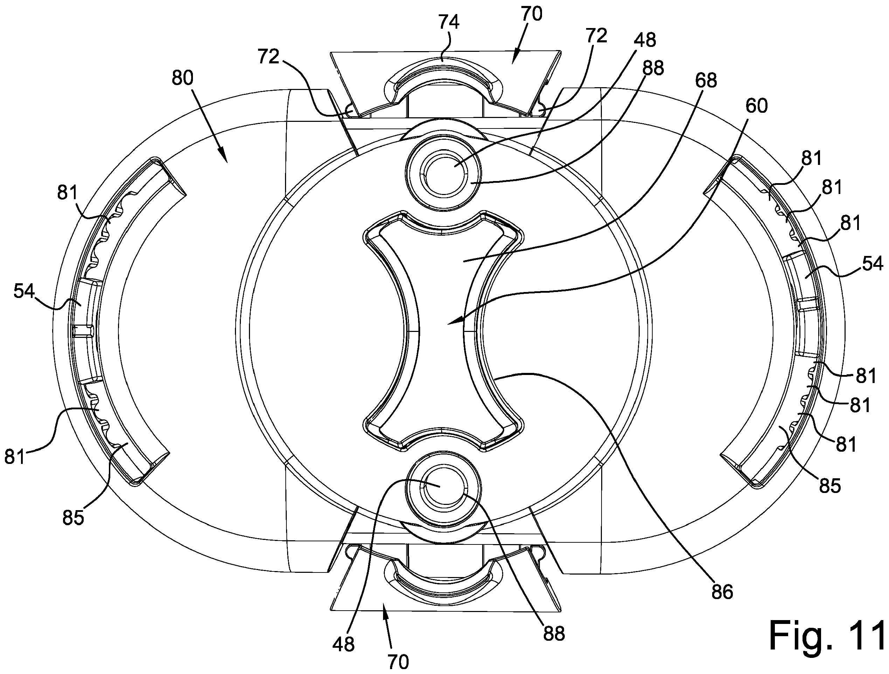

The outer face 61 of the toothed button 60 has a raised part 68 that is adapted to pass through a through opening 86 of the cover 80, as shown in Figure 11.

The engagement position of the toothed button 60 envisages that the elastic element is in the work position, that at least one part of the plurality of teeth 65 of the first side wall 63 of the toothed button 60 is engaged with at least one part of the plurality of teeth 56 of the first cam 50 and that at least one part of the plurality of teeth 65 of the second side wall 64 of the toothed button 60 is engaged with at least one part of the plurality of teeth 56 of the second cam 50.

The disengagement position of the toothed button 60 envisages that the elastic element 90 is in the elongated position, that not even one tooth 65 of the two side walls 63, 64 of the toothed button 60 is engaged with some of the teeth 56 of the two cams 50.

The plurality of teeth 56 of the cams 50 and the plurality of teeth 65 of the toothed button 60 advantageously allows the position of each cam 50 to be individually adjusted in the slot 29 of the arm 20, further allowing an end adjustment of the flexion-extension rotation width of the articulated rod 10 through a plurality of discrete angular positions.

The toothed button 60 also envisages two side walls 66 facing in the orthogonal direction with respect to the direction in which the two side walls 63 and 64 are facing. Each side wall 66 comprises two fins 67 that extend in a parallel plane to the rotation plane. Each fin 67 is adapted to be engaged by a stop element 76 of the stop button 70.

In the disengagement position the toothed button 60 has the fins 67 in abutment against an inner face of the cover 80 and the raised part 68 entering into the through opening 86 of the cover 80.

Advantageously the raised part 68 of the toothed button 60 engaged with the through opening 86 of the cover 80 allows the movement of the toothed button 60 to be guided more safely, at the same time facilitating the locking, unlocking and adjustment operations of the constraints to the flexion-extension rotation width of the articulated rod 10.

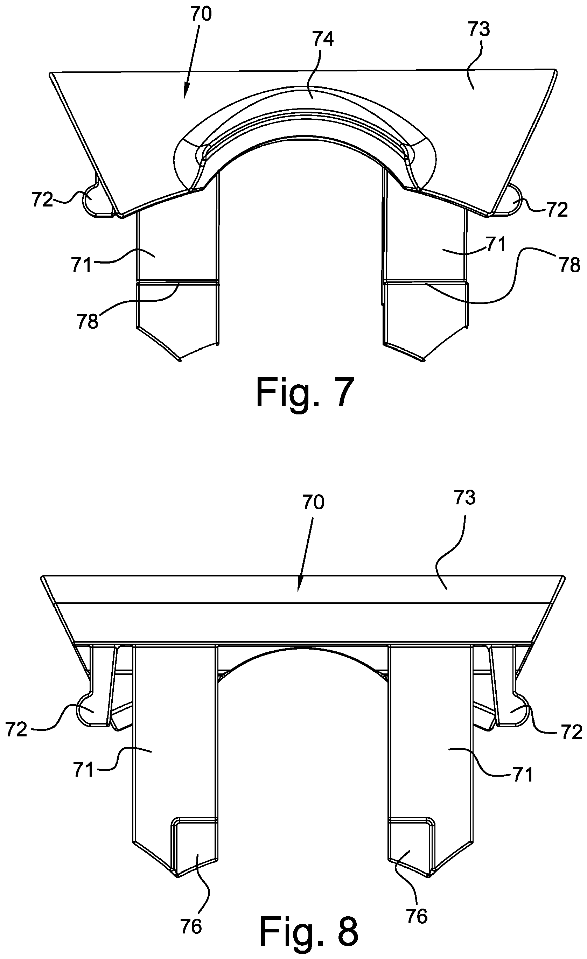

As shown in particular in Figures 7-8, the two stop buttons 70 are engaged with the cover 80 and are adapted to pass from a locking position to an unlocking position with the toothed button 60.

Each stop button 70 comprises a bridge 73 from which two extensions 71 project in the direction of the fins 67 of the toothed button 60. Each extension 71 envisages a seat 76 adapted to come into engagement with the respective fin 67 of the toothed button 60 for locking the toothed button 60 in the engagement position.

The seat 76 of the stop button 70 for the fin 67 of the toothed button 60 is provided on an inner face of the stop button 70.

The extension 71 comprises an end stop engagement 78 that is adapted to engage with a stroke end stop 87 of the cover 80 when the stop button 70 is in the unlocking position.

The outer face of the extension 71 bears the end stop engagement 78.

The extension 71 is sufficiently long to provide for the sliding of the stop button 70 from the locking position to the unlocking position.

The stop button 70 also envisages two elastic fastening elements 72 adapted to be engaged with respective seats 82 of the cover 80, so that in the locking position the two elastic fastening elements 72 are engaged in the seats 82 of the cover 80.

The stop button 70 further comprises a gripping raised part 74 that extends towards the outside.

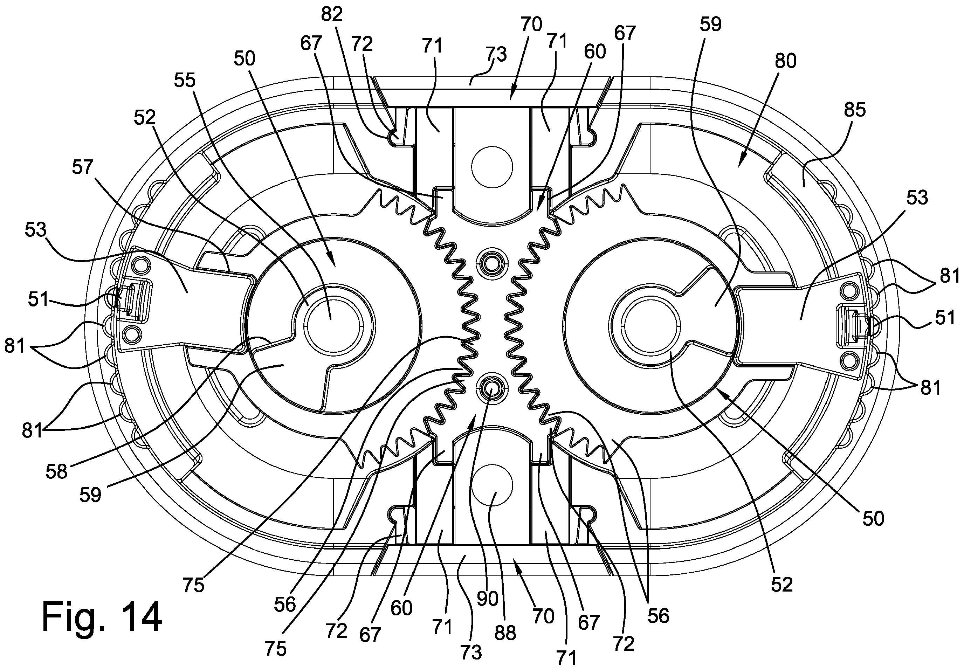

As shown in Figures 14-15, the locking position of the stop button 70 envisages that the two fastening elements 72 of the stop button 70 are engaged in the respective seats 82 of the cover 80, stop elements 76 of the stop button 70 are engaged above respective fins 67 of the toothed button 60 so that the toothed button 60 remains in the engagement position.

As shown in Figures 11-13, the unlocking position of the stop button 70 envisages that the fastening elements 72 of the stop button 70 are disengaged from the respective seats 82 of the cover 80, stop elements 76 of the stop button 70 are not engaged with the fins 67 of the toothed button 60 so that the toothed button 60 remains in the disengagement position.

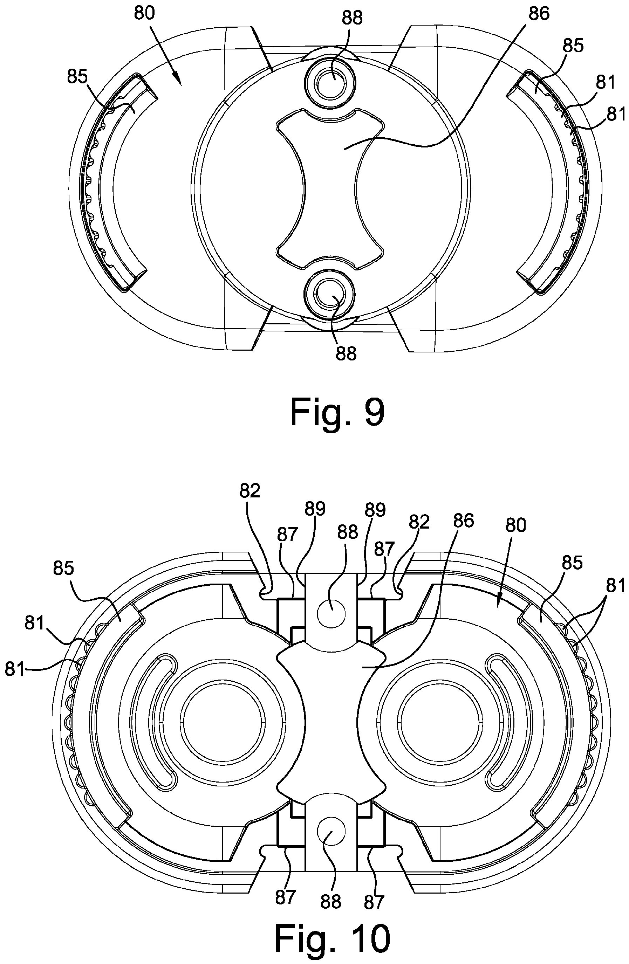

As shown in Figures 9-10, the cover 80 comprises four seats 82 for the respective four elastic fastening elements 72 of the stop button 70, two semi-circular slots 85 adapted to allow the passage of the indication slider 54 of the cam 50, the through opening 86 adapted to allow the passage of the raised part 68 of the toothed button 60, two through holes 88 adapted to engage two closing pins 48 that engage the cover 80 with the cover plate 30 by means of respective through holes 38 of the cover plate 30.

Each slot 85 of the cover 80 carries a plurality of seats 81 adapted to allow the engagement of the selecting tooth 51 of the cam 50.

The cover 80 bears a graduated scale, which is not shown in the figures, adapted to allow each position of the selecting tooth 51 in a seat 81 of the plurality of seats 81 of the cover 80 to match with a defined rotation width.

An inner face of the cover 80 comprises raised parts that are stroke end stops 87 adapted to stop the sliding of the extension 71 of the stop button 70 during the passage from the locking position to the unlocking position engaging the respective end stop engagements 78 of the stop button 70.

The cover 80 also envisages guide elements 89 adapted to allow the guided sliding of the extensions 71 of the stop button 70 while it passes from the locking position to the unlocking position.

In relation to the adjustment of the articulated rod 10, with particular reference to Figures 11-15 and starting from the unlocking position of the stop buttons 70, the orthopaedist constrains the flexion-extension rotation width by handling the two indication sliders 54 of the cams 50 that exit from the two slots 85 of the cover 80. In the unlocking position of the stop buttons 70 it is possible to independently adjust each cam 50 making it rotate by moving the indication slider 54 inside the slot 85 of the cover.

The cam 50 can rotate and is constrained by the abutment of the stop walls 57, 58 of the axial extension 59 of the cam 50 against the respective stop walls 27, 28 of the through slot 29 of the arm 20.

Once the position of the cams 50 has been chosen, the toothed button 60 is pressed from the outside towards the inside so that at least a part of the teeth 65 of the toothed button 60 are engaged with a respective plurality of teeth 56 of the cam 50, making the toothed button pass from the disengagement position to the engagement position.

After this step the stop buttons 70 are made to slide by the orthopaedist inside the cover 80 handling the gripping raised parts 74 of the stop button 70 and making the stop buttons 70 pass from the unlocking position to the locking position in which the stop elements 76 of the stop buttons 70 are positioned above the fins 67 of the toothed button 60 so as to lock the toothed button 60 in the engagement position, preventing the elastic elements 90 from passing to the elongated position. The elastic fastening elements 72 of the stop button 70 are engaged in the respective seats 82 of the cover 80 preventing the stop buttons 70 from passing to the unlocking position by mistake.

In relation to the operation of the articulated rod 10 after the orthopaedist has adjusted the position of the cams 50, the toothed button 60 is engaged with the two cams 50 and the stop button 70 is in the locking position, according to the preferred embodiment as shown in particular in Figure 2, each of the two cams 50 blocks the flexion and the other one the extension, respectively.

In relation to the extension rotation width, this is blocked by the stop wall 27 of the slot 29 that only abuts against the stop wall 57 of the first cam 50 and the two toothed ends 24 of the arms 20 are in abutment with each other.

In relation to the flexion rotation width, this is blocked by the stop wall 27 of the slot 29 that only abuts against the stop wall 57 of the second cam 50 and the two toothed ends 24 of the arms 20 are in abutment with each other. First cam 50 means the one shown on the left in Figure 2, whereas second cam 50 means the one shown on the right in Figure 2.

Advantageously, the articulated rod 10 of the present invention can be easily adjusted in flexion-extension, allowing each cam 50 to be adjusted independently, which is compact and small and is a lot safer.

Advantageously only one cam 50 and the block between the two toothed ends 24 constrains the flexion or extension rotation width. Advantageously there are no removable parts, hence making the adjustment of the articulated rod 10 safer and easier.

Alternatively, the slot 29 is separate from the through opening 35 and the cam 50 envisages the hollow axial extension 52 not being a single piece with the axial extension 59 adapted to be engaged in the slot 29 of the arm 20.

Also alternatively it is possible to envisage that the first stop wall 57 and the second stop wall 58 are not a single piece, i.e. that each cam 50 comprises two axial extensions 59, a first axial extension comprising the first stop wall 57 adapted to abut with the first end stop wall 27 of the slot 29 of the arm 20 and a second axial extension comprising the second stop wall 58 adapted to abut with the second stop wall 28 of the slot 29 of the arm 20.

A further alternative envisages that the radial extension 53 of the cam 50 is realized in a single piece with the cam 50.

Another alternative envisages that the toothed button 60 engages directly with engagement elements of the cover 80 without the need for stop buttons 70.

An alternative envisages that the stop buttons comprise engagement teeth with respective teeth 56 of the cams 50 so as to lock the position of the cams 50 in the locking position of the stop buttons.

More generally it is envisaged as an alternative that at least one locking element 60, 70 comprises a plurality of teeth that engage with the plurality of teeth 56 of the pair of cams 50 for constraining the rotation width of the articulated rod 10.

Also alternatively it is envisaged that the two slots 29 of the two arms 20 may not be arranged according to the central symmetry with respect to the geometric centre lying on the geometric plane of rotation. In said yet another alternative it is envisaged that different walls 57, 58 of the axial extension 59 can abut with the respective end stop walls 27, 28 of the slots 29.

Alternatively the indication slider 54 may be any other indication element adapted to determine the rotation position of the cam 50 with respect to the slot 29.

The articulated rod 10 according to the invention can be applied indifferently to orthopaedic devices for the upper or lower limbs.

The articulated rod thus conceived is susceptible to many modifications and variations, all falling within the invention; furthermore, all the details are replaceable by technically equivalent elements. In practice, the materials used, as well as the dimensions, can be any according to the technical requirements.

## Claims

1. Articulated rod (10) of an orthopaedic device comprising a pair of arms (20), two covering plates (30), a polycentric joint (40) that articulates said pair of arms (20) in mutual rotation, where said rotation comprises a direction of flexing rotation and a direction of extension rotation, two rotation pins (42) engaged in through holes (34) of at least one covering plate (32) of said two covering plates (30) and in through holes (25) of said pair of arms (20), **characterised in that** every arm (20) of said pair of arms (20) carries a slot (29), and **in that** the articulated rod (10) comprises a pair of cams (50), each cam (50) carrying a through hole (55) engaged by the rotation pin (42) and at least one covering plate (31) carrying two through openings (35), wherein each cam (50) is rotatably engaged in a respective through opening (35) of said two through openings (35) of said at least one covering plate (31), each cam (50) comprising at least one axial extension (59) adapted to be engaged in a respective slot (29) of the arm (20), each cam (50) comprising a plurality of coupling teeth (56) and an indication element (54), said articulated rod (10) also comprising at least one locking element (60, 70) comprising a plurality of teeth (65) adapted to be engaged with said plurality of coupling teeth (56) of the pair of cams (50) so as to constrain said rotation in at least one direction of rotation of said two directions of rotation in a preset angular width of the rotation.

2. Articulated rod (10) according to claim 1, **characterised in that** said at least one axial extension (59) of the cam (50) comprises two stop walls (57, 58), where at least one of said two stop walls (57, 58) is adapted to go into abutment with one of two end stop walls (27, 28) of said slot (29) of the arm (20).

3. Articulated rod (10) according to any one of claims 1 or 2, **characterised in that** said cam (50) comprises an outer portion and an inner portion overlapping one another on a geometric plane of rotation, the outer portion of the cam (50) comprises a first perimeter segment that comprises the plurality of coupling teeth (56), the inner portion of the cam (50) comprising a hollow axial extension (52) where the cavity defines the through hole (55) of the cam (50), where said hollow axial extension (52) comprises an outer disc adapted to be rotatably engaged with the through opening (35) of said at least one covering plate (31) and an inner disc adapted to be rotatably engaged with the through hole (25) of the arm (20).

4. Articulated rod (10) according to any one of claims 1-3, **characterised in that** the locking element (60, 70) is a toothed button (60) comprising two side walls (63, 64), each wall (63, 64) comprises a plurality of teeth (65) adapted to be engaged by at least one tooth (56) of the respective plurality of teeth (56) of a respective cam (50) of said pair of cams (50) when said locking element (60, 70) is in engagement position with said pair of cams (50).

5. Articulated rod (10) according to claim 4, **characterised in that** said toothed button (60) is mounted with said at least one plate (31) by means of at least one elastic element (90) adapted to pass said toothed button (60) from an engagement position to a disengagement position with said pair of cams (50), where said engagement position envisages that said at least one elastic element (90) is in work position, **in that** at least one tooth (65) of a first side wall (63) of the toothed button (60) is engaged with at least one tooth (56) of a first cam (50) and **in that** at least one tooth (65) of a second side wall (64) of the toothed button (60) is engaged with at least one tooth (56) of a second cam (50), where the disengagement position of the toothed button (60) entails that said at least one elastic element (90) is in elongated position, and that no tooth (65) of the two side walls (63, 64) of the toothed button (60) is engaged with any of the teeth (56) of the pair of cams (50).

6. Articulated rod (10) according to claim 5, **characterised in that** said toothed button (60) comprises at least one fin (67) adapted to be engaged with a stop element (76) of a stop button (70) when said toothed button (60) is in engagement position so as to lock the toothed button (60) in said engagement position.

7. Articulated rod (10) according to claim 6, **characterised in that** said stop button (70) is slidably associated with a cover (80) of said articulated rod (10) .

8. Articulated rod (10) according to any one of claims 6 or 7, **characterised in that** said stop button (70) comprises at least one elastic fastening element (72) adapted to be engaged with a respective seat (82) of the cover (80) keeping the stop button (70) in locking position and the toothed button (60) in engagement position with the pair of cams (50).

9. Articulated rod (10) according to any one of claims 1-8, **characterised in that** said cover (80) carries a pair of slots (85) adapted to engage said indication element (54) of the respective cam (50).

10. Articulated rod (10) according to claim 9, **characterised in that** each slot (85) of said cover (80) carries a plurality of seats (81) adapted to allow the engagement of a selecting tooth (51) of said cam (50) that is at said indication element (54).
